# EUROPEAN PATENT SPECIFICATION

(11) **EP 4 066 818 B1**
(45) Date of publication and mention of the grant of the patent: **29.11.2023**
(21) Application number: 21166554.2
(22) Date of filing: 01.04.2021
(51) Int. Cl.: C08L 67/04, A61K 9/51, C08K 5/3462, A61K 49/00, A61K 41/00

(54) **NEAR INFRARED ABSORBING COMPOSITE RESIN PARTICLES**
NAH-INFRAROT-ABSORBIERENDE VERBUNDHARZTEILCHEN
PARTICULES DE RÉSINE COMPOSITE ABSORBANT LE PROCHE INFRAROUGE

(43) Date of publication of application: 05.10.2022
(73) Proprietor: AGFA-GEVAERT NV, 2640 Mortsel (BE)
(72) Inventor: LOCCUFIER, Johan, 2640 Mortsel (BE); LOUWET, Jos, 2640 Mortsel (BE)
(74) Representative: Lingier, Stefaan Frans L.

(56) References cited:
- WO-A1-2020/011601
- WO-A2-2020/172618
- ZHANG YUANYUAN ET AL: "Polymeric nanocarriers incorporating near-infrared absorbing agents for potent photothermal therapy of cancer", POLYMER JOURNAL, vol. 48, no. 5, 16 December 2015 (2015-12-16), pages 589-603, XP037325114, ISSN: 0032-3896, DOI: 10.1038/PJ.2015.117
- DUONG TONY ET AL: "Phototheranostic nanoplatform based on a single cyanine dye for image-guided combinatorial phototherapy", NANOMEDICINE , NANOTECHNOLOGY , BIOLOGY AND MEDICINE, vol. 13, no. 3, 1 April 2017 (2017-04-01), pages 955-963, XP029977657, ISSN: 1549-9634, DOI: 10.1016/J.NANO.2016.11.005

## Description

### Technical Field

The present invention relates to a biocompatible organic nano- and microparticle design for opto-medical applications such as phototherapies including photothermal therapy (PTT), photodynamic therapy (PDT), chemo-photodynamic therapy and fluorescence medical imaging.

### Background Art

Near infrared (NIR) laser technology is gaining importance in non-invasive treatment of different diseases and in medical diagnostics, including photothermal therapy, photodynamic therapy, fluorescence imaging and photo-acoustic imaging. Several of these technologies rely on NIR absorbing nanoparticles, where often inorganic nanoparticles are used, such as gold nanomaterials, carbon nanomaterials, including carbon nanotubes, metal sulfides, metal oxides and different upconverting nanoparticles. The use of inorganic optothermal converting nanoparticles has extensively been reviewed (Raza et al., Journal of Materials Research and Technology, 8(1), 1497-1509 (2019); Wang et al., International Journal of Nanomedicine, 15, 1903-1914 (2020)). Although giving excellent NIR response, these nanoparticles are not biodegradable and hold the risk for bioaccumulation and long retention time in the body that could potentially increase their probability of long term toxicity. Therefore, nanoparticles based on biocompatible organic NIR absorbers would be highly preferred.

Although several classical organic NIR absorbers are well documented in opto-medical applications, cyanine dyes are a particularly preferred class of NIR absorbers due to their high molar extinction coefficient. High molar extinction of NIR laser light has the advantage that the amount of required NIR absorber can be reduced and makes application of in vitro and in vivo imaging and treatment of deeply-sited diseases such as tumours, possible. One of the best known and also FDA approved cyanine dye, is indocyanine green.

NIR absorbers such as indocyanine green have to be integrated in nanoparticles to increase their lifetime in the body, be dispersible in the aqueous fluids of the body, prevent photo-bleaching and increase its tumour targeting ability.

Integrating NIR absorbers into nanoparticles have furthermore other additional benefits. It is known that the spectral characteristics of several classes of NIR absorbers are very dependent on their environment and can change in function of e.g. pH and ionic strength, caused by aggregation phenomena. Therefore, physically integrating NIR absorbers into a nanoparticle makes the spectral characteristic and photophysics of the absorber independent of the external environment. The response in a physiological environment becomes then very predictable. Tuning the laser response is simply done by adapting the concentration of the NIR absorber in the nanoparticle, avoiding laborious synthesis of NIR absorbers each time.

Several designs of nanoparticles have been proposed in the prior art and recently reviewed (Zhu et al. Biomater. Sci, 6, 746-765 (2018); Zhu et al., Current Medicinal Chemistry, 26, 1389-1405 (2019); Hg. Et al., Chemical reviews, 115, 11012-11042 (2015); Shao et al., RSC Nanoscience & Nanotechnology, 40, 125-157 (2016)). Proposed nanoparticle designs often require laborious synthetic protocols, hampering easy tuning of the nanoparticles towards different applications. Therefore, there is a need for near infrared responsive nanoparticles that are accessible via simple and scalable industrial technologies. One of these simple and scalable industrial technologies, is solvent evaporation. DUONG TONY ET AL ( "Phototheranostic nanoplatform based on a single cyanine dye for image-guided combinatorial phototherapy) ,NANOMEDICINE , NANOTECHNOLOGY , BIOLOGY AND MEDICINE, vol. 13, no. 3 , pages 955-963,discloses nanoparticles based on PEG-PCL as polymeric carrier and the same NIR dye (IR 775 and IR 797).

Cyanine dyes, such as indocyanine green are however, often not compatible with industrial and easy scalable technologies such as solvent evaporation to physically integrate them into biocompatible nanoparticles. Indocyanine green is not or very limited soluble in the solvents currently used in solvent evaporation techniques. Due to its partial water solubility and degradation in water, the indocyanin green dyes have to be completely encapsulated to avoid any contact with water. This requirement further limits the availability of techniques for making fully encapsulated indocyanin green nano- and microparticles.

Hence there is a need to integrate organic NIR absorbers with a high molar extinction coefficient in biocompatible nanoparticles, using industrial solvent evaporation technologies without the need of fully encapsulating the NIR absorbers.

### Summary of invention

It is an object of the invention to provide a specific class of NIR absorbers which can be integrated with biocompatible polymers via industrial and easy scalable technologies into composite resin particles as defined in Claim 1.

It is an even further aspect of the present invention to provide nano- or microcapsules of biocompatible polymers containing specific non-ionic NIR absorbers as defined in Claim 4.

It is a further aspect of the present invention to provide an aqueous dispersion of the composite resin particles as defined in Claim 1. The aqueous dispersion is defined in Claim 8

According to another aspect, the present invention includes an industrial scalable method of integrating the specific class of NIR absorbers with biocompatible polymers as defined in Claim 14

Other features, elements, steps, characteristics and advantages of the present invention will become more apparent from the following detailed description of preferred embodiments of the present invention. Specific embodiments of the invention are also defined in the dependent claims.

### Description of embodiments

The objects of the present invention are realized by composite resin particles comprising a NIR absorber and a biocompatible resin. The NIR absorber is a compound according to general formula I (see below).

### A. The composite resin particle

### A.1. The NIR absorber

The NIR absorber present in the composite resin particles of the invention is according general formula I 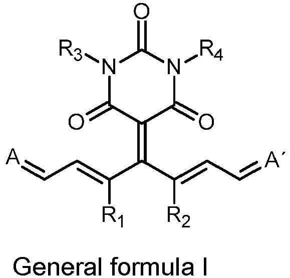 wherein
A and A' independently represent a substituted or unsubstituted heterocyclic group, covalently bonded to the polymethine chromophore via a carbon atom
R₁ and R₂ are independently selected from the group consisting of a hydrogen, a substituted or unsubstituted alkyl group, a substituted or unsubstituted alkenyl group, a substituted or unsubstituted alkynyl group, a substituted or unsubstituted aralkyl group, a substituted or unsubstituted alkaryl group and a substituted or unsubstituted aryl or heteroaryl group R₁ and R₂ may represent the necessary atoms to form a five to eight membered ring
R₃ and R₄ are independently selected from the group consisting of a substituted or unsubstituted alkyl group, a substituted or unsubstituted alkenyl group, a substituted or unsubstituted alkynyl group, a substituted or unsubstituted aralkyl group, a substituted or unsubstituted alkaryl group and a substituted or unsubstituted aryl or heteroaryl group.

In a preferred embodiment, said NIR absorber represents a compound according to general formula II: 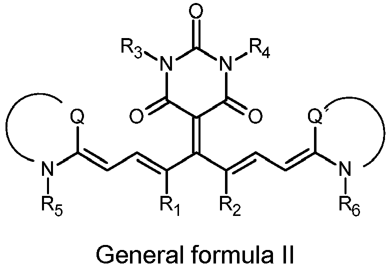 wherein,
R₁ and R₂ are independently selected from the group consisting of a hydrogen, a substituted or unsubstituted alkyl group, a substituted or unsubstituted alkenyl group, a substituted or unsubstituted alkynyl group, a substituted or unsubstituted aralkyl group, a substituted or unsubstituted alkaryl group and a substituted or unsubstituted aryl or heteroaryl group R₁ and R₂ may represent the necessary atoms to form a five to eight membered ring
R₃ and R₄ are independently selected from the group consisting of a substituted or unsubstituted alkyl group, a substituted or unsubstituted alkenyl group, a substituted or unsubstituted alkynyl group, a substituted or unsubstituted aralkyl group, a substituted or unsubstituted alkaryl group and a substituted or unsubstituted aryl or heteroaryl group R₅ and R₆ independently represent a substituted or unsubstituted alkyl group, a substituted or unsubstituted alkenyl group, a substituted or unsubstituted alkynyl group, a substituted or unsubstituted aralkyl group, a substituted or unsubstituted alkaryl group and a substituted or unsubstituted aryl or heteroaryl group
Q represents the necessary atoms to form a substituted or unsubstituted five or six membered heteroring

In a further preferred embodiment, R₁ and R₂ represent the necessary atoms to form a substituted or unsubstituted five or six membered ring, a six membered ring being the most preferred.

In another preferred embodiment, R₃ and R₄ independently represent a substituted or unsubstituted alkyl group, a substituted or unsubstituted alkenyl group, a substituted or unsubstituted alkynyl group and a substituted or unsubstituted aralkyl group, a substituted or unsubstituted alkyl group being more preferred.

In a further preferred embodiment, A and A' independently are selected from the group consisting of a substituted or unsubstituted indolinine, a substituted or unsubstituted naphtinolinine, a substituted or unsubstituted naphtostyryl group, a substituted or unsubstituted benzimidazole, a substituted or unsubstituted benzothiazole, a substituted or unsubstituted benzoxazole, a substituted or unsubstituted pyridine and a substituted or unsubstituted quinolone. Indolinines, naphtindolinines and naphtostyryls are particularly preferred.

In an even further preferred embodiment, at least one and more preferably at least two of R₃, R₄, R₅ and R₆ represent a branched substituted or unsubstituted alkyl group.

A branched alkyl group is defined as an alkyl group wherein at least a second group selected from the group consisting of an alkyl group, an alkenyl group, an alkynyl group, an aralkyl group, an alkaryl group and an aryl or heteroaryl group is substituted on a non-terminal carbon atom of the alkyl chain. Most preferably, said branched alkyl group is substituted by an alkyl group.

Typical examples of NIR absorbers according to the present invention are given in Table 1 without being limited thereto.

**Table 1**

| | |
|---|---|
| 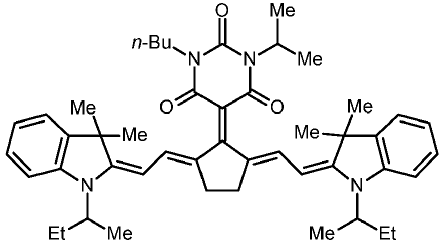 | NIR-1 |
| 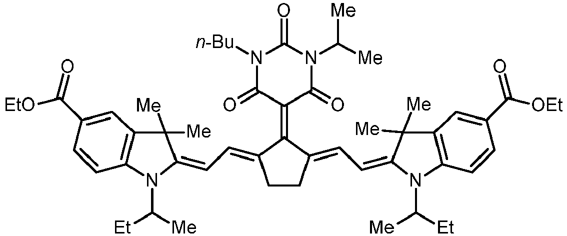 | NIR-2 |
| 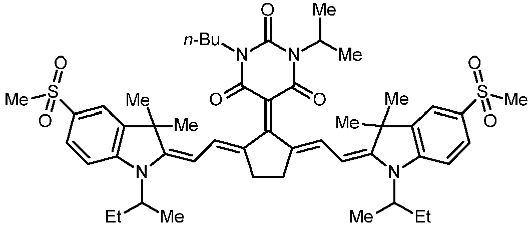 | NIR-3 |
| 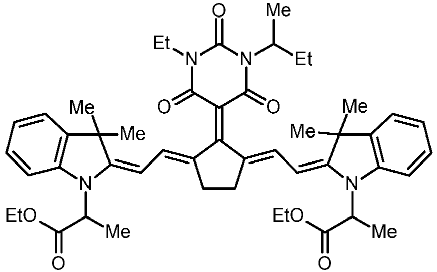 | NIR-4 |
| 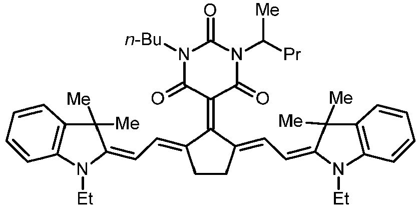 | NIR-5 |
| 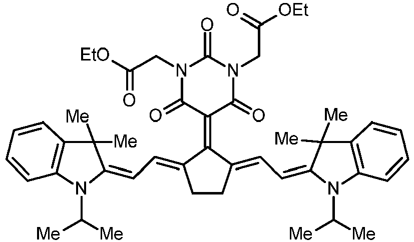 | NIR-6 |
| 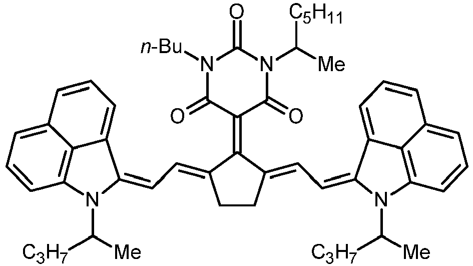 | NIR-7 |
| 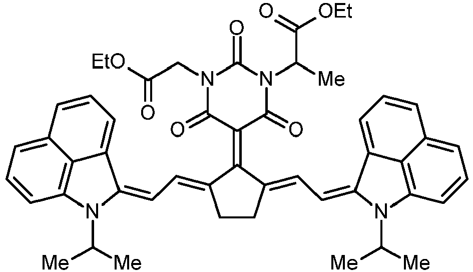 | NIR-8 |
| 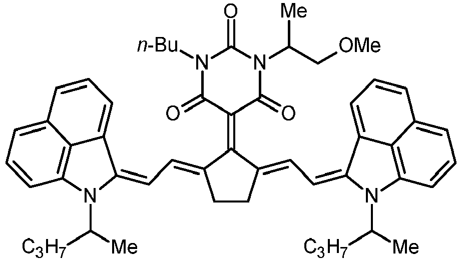 | NIR-9 |
| 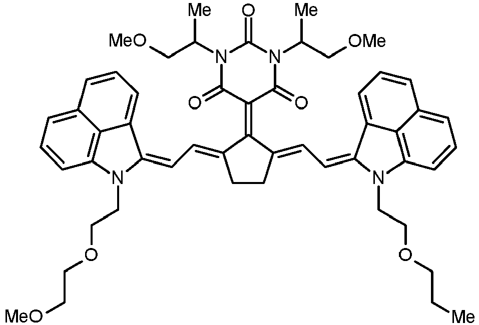 | NIR-10 |
| 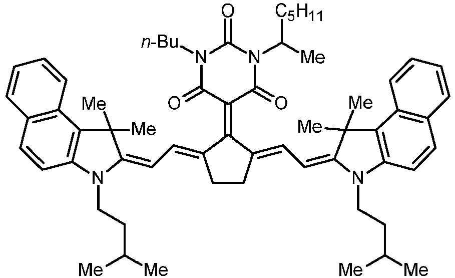 | NIR-11 |
| 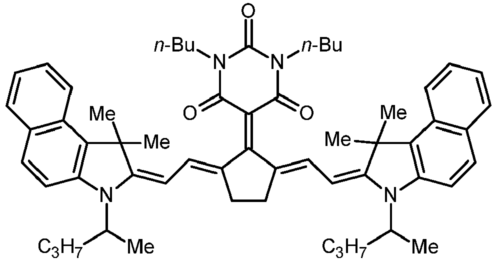 | NIR-12 |
| 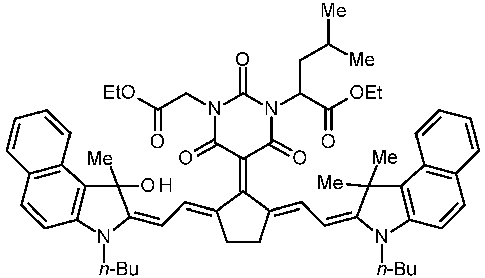 | NIR-13 |
| 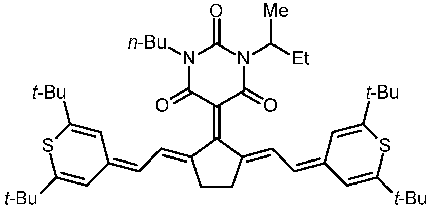 | NIR-14 |
| 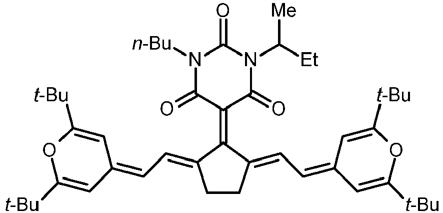 | NIR-15 |
| 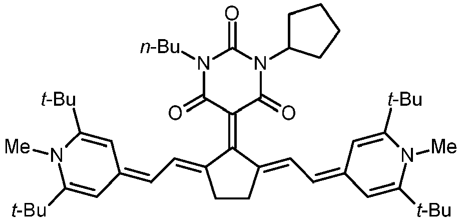 | NIR-16 |
| 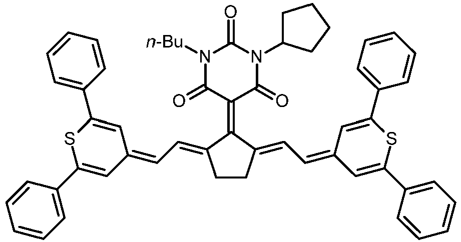 | NIR-17 |
| 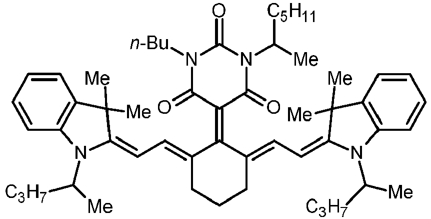 | NIR-18 |
| 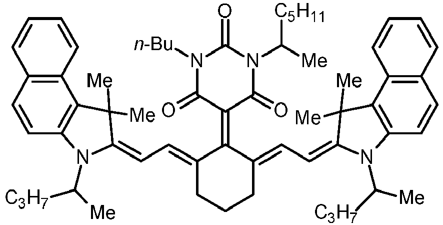 | NIR-19 |
| 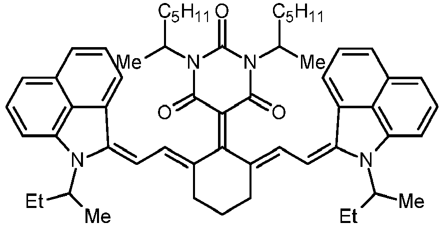 | NIR-20 |
| 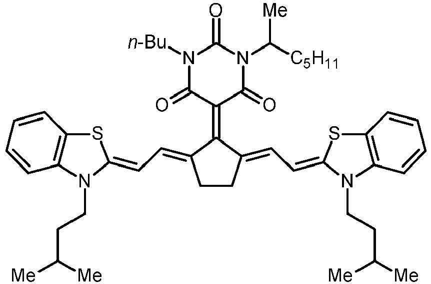 | NIR-21 |
| 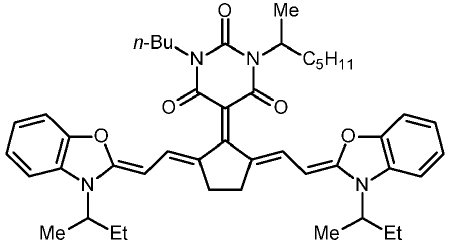 | NIR-22 |
| 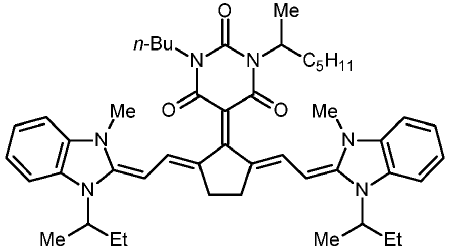 | NIR-23 |
| 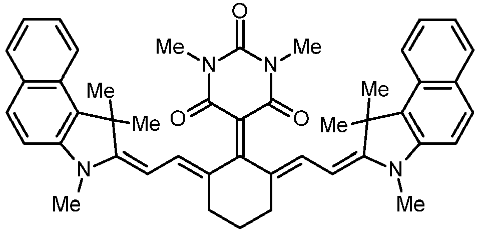 | NIR-24 |
| 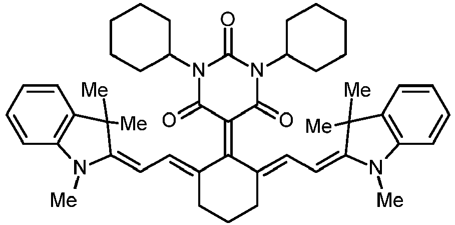 | NIR-25 |

The NIR absorber preferably has an absorption maximum between 700 and 1200 nm, more preferably between 750 and 1150 nm and most preferably between 780 and 1100 nm.

In a preferred embodiment of the invention, the NIR absorber is non-ionic. Due to its non-ionic character, a high solubility in solvents used in solvent evaporation technologies is obtained. Hence, high concentration of NIR absorbers can be obtained in the resin particle. Non-ionic NIR absorbers also show a low water solubility. Due to the low water solubility, no complete encapsulation by the resin in the composite resin particle is required.

When the composite resin particles are dispersed in an aqueous vehicle, the NIR absorber content in the dispersion is preferably between 0.05 wt.% to 15 w% on the total solid content of the dispersion, more preferably between 0.1 w% and 10 w% and most preferably between 0.25 w% and 5 w%.

### A.2. The biocompatible resin

The biocompatible resin present in the composite resin particle according to the present invention is preferably soluble in a substantially water immiscible solvent. A substantially water immiscible solvent is defined as a solvent that forms a two phase system at room temperature when mixed with water in a one to one ratio. Esters and ketones are particularly preferred water immiscible solvents.

Biodegradable and biocompatible resins can be selected from the resins as discussed in Biodegradable Polymers, PBM Series, Volume 2 (Citus Books 2003, ISSN 1479-1285). The resin according to the present invention is selected from the group consisting of (poly(amino acids), polyphosphazenes, polysaccharide derivatives, poly(esters), poly(ortho esters), poly(cyano-acrylates) and copolymers thereof, poly(amino acids) and poly(esters) being more preferred.

The poly(ester) is preferably a poly(caprolactone), a poly(lactic acid), a poly(L-lactic acid), a poly(glycolic acid), a poly(lactic acid-co-glycolic acid), a poly(L-lactic acid-co-glycolicacid), a poly(D, L-lactide) and any derivative and/or combination thereof.

### A.3. Pharmaceutical active compound

The composite resin particles are also suitable for on-demand drug release wherein the drug is released upon heating the particles by means of an appropriate NIR light source such as a NIR laser. Therefor it is useful to incorporate a pharmaceutical compound to achieve this on-demand drug release.

Sometimes, PTT or PDT cannot completely destruct cancer cells and may result in the survival of the residual cells after photothermal treatment. Therefor it is useful to incorporate anti-cancer drugs for enhanced chemotherapy. The drug will be released upon application of NIR light on the composite particle due to the heat generated, triggering synergetic chemo-photothermal therapy. The anti-cancer drug should preferably be soluble in the water immiscible solvent used in the preparation of the composite resin particles (see § B.).

Anti-cancer drugs which are suitable to be incorporated in the particles of the invention are cytostatics. Cytostatics for the treatment of cancer can be selected from the group consisting of alkylating agents, anthracyclines, cytoskeletal disruptors, epothilones, histone deacetylase inhibitors, topoisomerase I inhibitors, topoisomerase II inhibitors, kinase inhibitors, nucleotide analogs, peptide antibiotics, platinum based agents, retinoids and vinca alkaloids and derivatives. Alkylating agents can be bi- or monofunctional. Typical bifunctional alkylating agents are cyclophosphamide, mechlorethamine, chlorambucil and melphalan. Typical monofunctional alkylating agents are dacarbazine, nitrosoureas and temozolomide. Typical anthracyclines are daunorubicin, doxorubicin, epirubicin, idarubicin, mitoxantrone and valrubicin. Typical cytoskeletal disruptors are paclitaxel, docetaxel, abraxane and taxotere. Typical histone deacetylase inhibitors are vorinostat and romidepsin. Typical topoisomerase I inhibitors are irinotecan and topotecan. Typical topoisomerase II inhibitors are etoposide, teniposide and tafluposide. Typical kinase inhibitors are bortezomib, erlotinib, gefitinib, imatinib, vemurafenib and vismodegib. Typical nucleotide analogs are azathioprine, capecitabine, cytarabine, doxifluridine, fluorouracil, gemcitabine, hydroxyurea, mercaptopurine, methotrexate and tioguanine. Typical retinoids are tretinoin, alitretinoin and bexarotene. Typical vinca alkaloids are vinblastine, vincristine and vindesine.

### B. Preparation of composite resin particle and composite particle dispersion

The composite resin particle according to the present invention is a mixture of a biocompatible resin which is preferably soluble in a water immiscible solvent and selected from the group consisting of (poly(amino acids), polyphosphazenes, polysaccharide derivatives, poly(esters), poly(ortho esters), poly(cyano-acrylates) and copolymers thereof, poly(amino acids) and poly(esters) and at least one NIR infrared dye according to the present invention (see §A.1).

The resins according to the present invention can be functionalized with functional groups making the resin self-dispersing in water, with the proviso that the functionalization is limited to avoid that the resin becomes fully water soluble. Typical functional groups making resins self-dispersing are selected from the group consisting of a poly(ethylene oxide), a carboxylic acid or salt thereof, a sulfonic acid or salt thereof, a phosphonic acid or salt thereof, a phosphoric acid monoester or salt thereof, a sulfuric acid monoester or salt thereof, an ammonium group, a sulfonium group and a phosphonium group.

The incorporation of a polyethylene gycol) functional group is particularly useful to give stealth properties to the composite resin particles of the invention in the human or animal body. These stealth properties are required to avoid fast uptake by the reticuloendothelial system and only being uphold at the required site.

In a preferred embodiment, the resin according to the present invention is not functionalized with a functional group making the resin self-dispersing.

The use of a dispersant in the preparation of an aqueous dispersion of the composite resin particles is particularly preferred. This allows to adjust the surface characteristics without the need for designing specialty resins. Standard biocompatible resins can be used in the preparation of the resin particles. The dispersant can be a surfactant or a stabilizing polymer. The surfactant can be non-ionic, anionic, cationic or zwitterionic. Non-ionic ethoxylated block copolymer surfactants are preferred. EO-PPO-EO triblock copolymers even more preferred. The block copolymers give the particles of the invention stealth properties but also lead to smaller particle diameters. As stabilizing polymers, hydroxyl functionalized polymers are particularly preferred, preferably selected from polysaccharides and poly(vinyl alcohol) or poly(vinyl alcohol) copolymers or derivatives thereof.

In a preferred embodiment, the particle size of the composite resin particles according to the present invention is between 0.03 µm and 3 µm, more preferably between 0.05 µm and 1 µm and most preferably between 0.07 µm and 0.5 µm. Composite resin particles according to the present invention having a particle size below 0.25 µm are particularly preferred.

A particularly preferred method for the preparation of composite resin particle dispersion according to the present invention includes the following steps:
1. Dissolving the resin according to the present invention and the NIR absorber according to the present invention in a water immiscible solvent; and
2. Dissolving a dispersant into water; and
3. Emulsifying the solution of the resin and the NIR absorber into the aqueous solution of the dispersant; and
4. Evaporating the water immiscible solvent.

The composite resin particles in dried state if required, can be obtained by isolating the composite particles via a separation technique such as centrifuge, freeze-drying, ...

If an additional functional compound such as a pharmaceutical active agent has to be integrated in the composite resin particle, this compound is preferably dissolved in the water immiscible solvent. The solvent evaporation technology according to the present invention is particularly of interest for the incorporation of active pharmaceutical ingredients such as anti-cancer drugs.

A particularly preferred method for the preparation of a dispersion of the composite resin particles according to the present invention, comprising an additional functional compound such as an active pharmaceutical ingredient includes the following steps:
1. Dissolving the resin according to the present invention, the NIR absorber according to the present invention and a functional compound in a substantially water immiscible solvent; and
2. Dissolving the dispersant into water; and
3. Emulsifying the solution of the resin, the NIR absorber and the functional compound into the aqueous solution of the dispersant; and
4. Evaporating the substantially water immiscible solvent.

The composite resin particles can be obtained the same way as described above.

### C. Fields of Application

The composite resin particles according to the invention are suitable for imaging affected organs in the human and/or animal body. The strong absorption of NIR light makes them also suitable for diffuse optical tomography and photoacoustic imaging.

When irradiated with an appropriate NIR laser, the NIR absorber in the composite particle of the invention can convert the absorbed photon energy into heat, directly ablating cancer cells with minimal invasion to surrounding healthy tissues making the particles very suitable in tumour phototherapy treatment (PTT).

The composite resin particle of the invention is also useful in photodynamic therapy (PDT) wherein the NIR absorber is excited with light of an appropriate wavelength for converting molecular oxygen into cytotoxic reactive oxygen species (ROS), such as singlet oxygen, which in turn damages cancer cells through oxidative stress and consequently induces cell death.

### D. Examples

### D.1. Materials:

- **Mowiol 4 88** is a poly(vinyl alcohol) supplied by Kuraray.
- **Synperonic PE/F68-FL** is a PEO-PPO-block copolymer surfactant supplied by Croda Chemicals International Ltd.
- **Natureworks Polylactide Resin 4060D** is a poly(lactide) supplied by Evans Chemetics.
- **Resomer RG503H** is a poly(D,L-lactide-co-glycolide) supplied by Evonik Industries.
- **NIR-7 is a NIR absorber and is prepared as follows:** 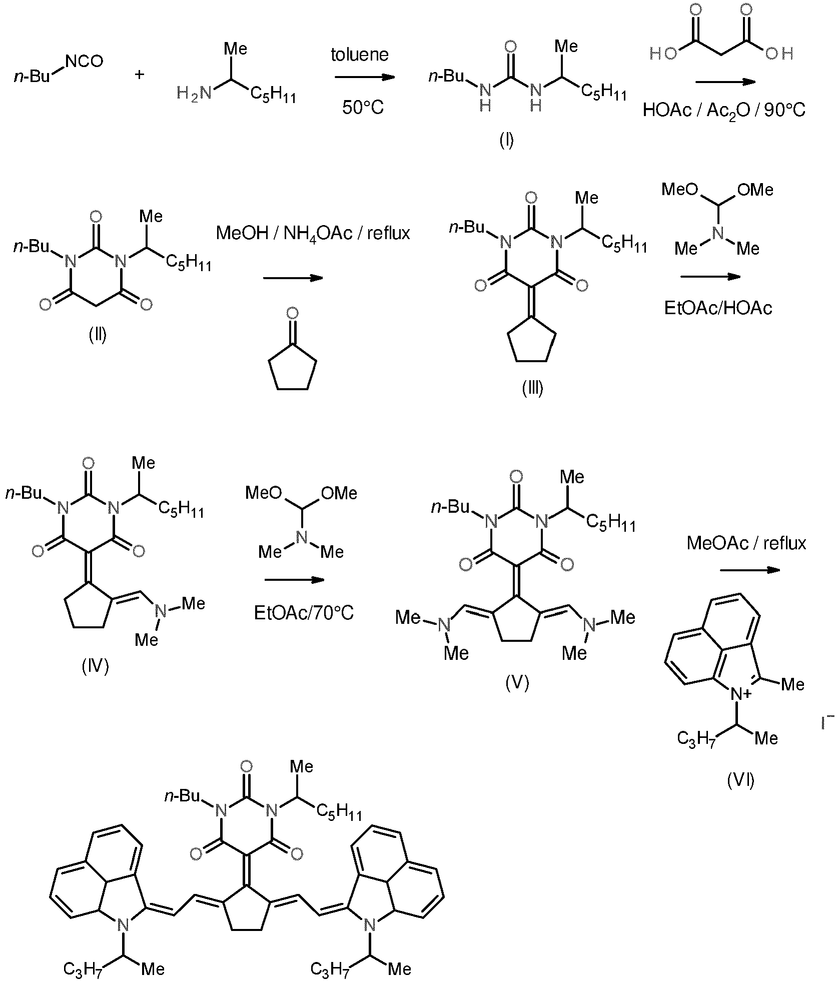
   - The synthesis of the ureum (I) 146 g n.-butyl isocyanate was dissolved in 85 ml toluene. 166 g 2-heptyl amine was added over two hours while the temperature was kept below 50 °C. The reaction was allowed to continue for 30 minutes at 50°C. The solvent and the excess of n.-butyl isocyanate were removed under reduced pressure and the crude ureum was used in the second step without further purification.
   - The synthesis of the barbiturate (II) 206 g acetic acid was added to 300 g of the ureum (I). The mixture was heated to 60°C. The solution of ureum (I) in acetic acid was added to 147 g malonic acid at 60°C. This solution was added to 292 g acetic anhydride. The reaction mixture was gently heated to 90°C and the reaction was allowed to continue for two and a half hours at 90°C. The reaction was allowed to cool down to 50°C and 78 g methanol was added. The mixture was refluxed for 45 minutes. The mixture was allowed to cool down to room temperature and the solvent was evaporated under reduced pressure. The residue was redissolved in 311 g methyl t.butyl ether and extracted three times with 2010 g of a 5w% sodium chloride solution. The solvent was removed under reduced pressure. Four times 35 ml toluene was added followed by removal under reduced pressure. The crude barbituric acid derivative (II) was used without further purification.
   - The synthesis of intermediate (III) 84 g cyclopentanone was added to 240 g of the barbituric acid derivative (II). 5 g ammonium acetate was added followed by the addition of 101 g methanol. The reaction mixture was heated to reflux and the reaction was allowed to continue for four and a half hours at reflux. The reaction mixture was allowed to cool down to room temperature and the solvent was removed at 50 mbar pressure and 95°C. Four times 5 ml toluene was added followed by evaporation at 50 mbar and 100°C. The reaction mixture was allowed to cool down to room temperature and 92 g toluene was added. 26 g silicagel in 55 g toluene was added and the mixture was filtered. The silicagel was flushed with toluene. The pooled toluene fractions were treated twice with 26 g silicagel in 55 g toluene, filtered and followed by flushing the silicagel with toluene. All toluene fractions were pooled, followed by evaporation of the solvent under reduced pressure. 287 g (y: 97%) of the crude intermediate (III) was isolated.
   - The synthesis of intermediate (V): 0.685 kg of intermediate (III) was dissolved in 0.334 kg ethyl acetate. The solution was cooled to 10°C and 18.9 g acetic acid was added. 0.273 kg N,N-dimethylformamide dimethyl acetal was added over 10 minutes, while the temperature rose to 20°C. The reaction was allowed to continue for 30 minutes at room temperature. The reaction mixture was heated to 45°C and 0.604 kg dimethylformamide dimethyl acetal was added over 15 minutes, followed by heating the reaction mixture to 65°C. The reaction was allowed to continue for 25 minutes at 65°C. The reaction mixture was allowed to cool to 47°C, followed by the addition of 1.06 kg methyl t.-butyl ether and 1.61 kg n.-hexane. The reaction mixture was cooled to 7°C. The crystallized intermediate V was isolated by filtration, washed with 160 g ethyl acetate and 60 g methyl t.-butyl ether, followed by three times washing with 160 g ethyl acetate and 60 g n.-hexane and once with 400 g heptane. The isolated intermediate (V) was dried. 335 g (y: 37%) of intermediate (V) was isolated.
   - The synthesis of intermediate (VI) : The intermediate (VI) can be prepared as disclosed in WO2013037672.
   - The synthesis of NIR-7: 621 g of intermediate (VI) was dissolved in 3.2 I methyl acetate. The reaction mixture was heated to 40°C. 372 g of intermediate (V) was added and the reaction was allowed to continue for two and a half hours at 50°C. The reaction mixture is cooled to 20°C. The crystallized NIR-7 was isolated by filtration, washed with 284 ml methyl acetate, 2.84 I ethyl acetate and 530 ml methyl t.-butyl ether. The crude NIR-7 was treated in 2.9 L water, isolated by filtration, washed with 1.5 I water, 142 ml methyl acetate, 280 ml ethyl acetate and 800 ml methyl t.-butyl ether and dried. 597 g (y : 87%) of NIR-7 was isolated.
- **NIR-24** is a NIR absorber and is prepared as follows: 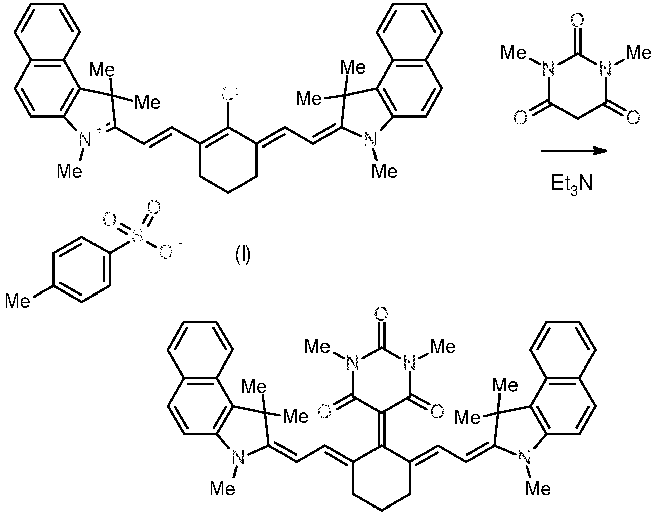 The starting NIR dye (I) can be prepared as disclosed by Nagao et al. (Dyes and Pigments, 73(3), 344-352 (2006).
   - The synthesis of NIR-24 30 g of NIR starting material (I) was added to 100 ml acetonitrile. 6.24 g N,N'-dimethyl barbituric acid was added, followed by the addition of 5.5 ml (4.0 g) triethyl amine. The reaction was allowed to continue for three hours at room temperature. The crude NIR-24 was isolated by filtration and treated in methanol at reflux. NIR-24 was isolated by filtration of the warm methanol solution and dried. 19.7 g (y : 35%) of NIR-24 was isolated.
- NIR-25 is a NIR absorber and is prepared as follows: 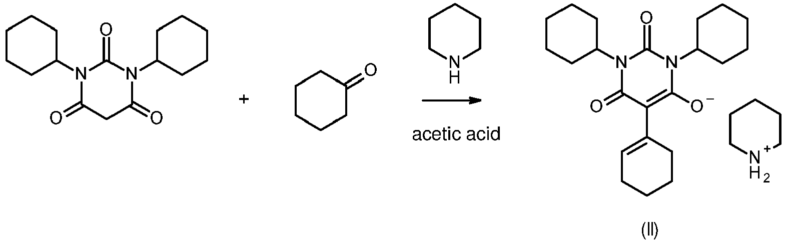 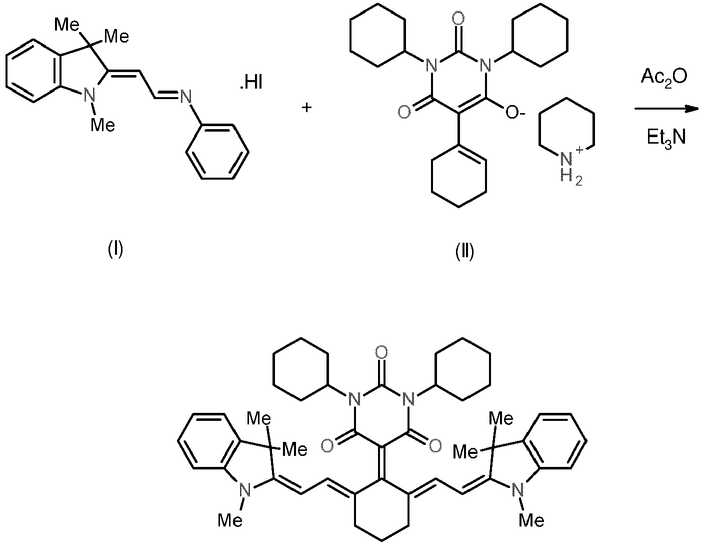
   - The synthesis of intermediate (I) :
      Intermediate (I) can be prepared as disclosed in WO2010120058.
   - The synthesis of intermediate (II) :
      292 g N,N'-dicyclohexyl barbituric acid was dissolved in 1.5 liter trichloroethane. 156 ml cyclohexanone, 12 ml (0.12 mol) piperidine and 15 ml acetic acid were added and the reaction mixture was heated to reflux. Water was removed by azeotrpic distillation using a Dean-Stark trap. The reaction was allowed to continue for 24 hours. The reaction mixture was allowed to cool down to room temperature and the solvent was removed under reduced pressure. The residue was redissolved in 1 liter trichloroethane. The undissolved residues were removed by filtration. 100 ml piperidine was added and intermediate (II) crystallized from the medium. Intermediate (II) was isolated by filtration and dried. 302 g (y : 67%) was isolated.
   - The synthesis of NIR-25 :

301.6 g of intermediate (II) and 533 g of intermediate (I) were dissolved in 1.05 I of N,N'-dimethyl imidazolone. 186 ml acetic anhydride and 366 ml triethyl amine were added and the reaction mixture was heated to 100°C. The reaction was allowed to continue for 30 minutes at 100°C. The reaction mixture was allowed to cool down to room temperature and NIR-25 was allowed to crystallize from the reaction mixture. The crude NIR-25 was isolated by filtration and treated with 1.3 I acetone followed by treating with 1.3 I methyl t.butyl ether. The isolated NIR-25 was redissolved in a 1/1 mixture of dichloromethane and methanol. Residual impurities were removed by filtration. 6.6 I methyl t.butyl ether was added and NIR-25 was allowed to crystallize from the medium. NIR-25 was isolated by filtration and dried. 136 g (y: 28%) of NIR-25 was isolated.

### 0.2. Methods

The particle size of the capsules was measured using a ZetasizerTM Nano-S (Malvern Instruments, Goffin Meyvis).

The UV-VIS spectra were measured on an Agilent 8433 spectrophotometer for spectra up to 1100 nm. The more bathochromic NIR absorbers were measured on a Shimadzu UV2600 spectrophotometer. The absorption of the samples at λₘₐₓ was adjusted to 1 by diluting the samples with water.

### 0.3. Example 1

This example illustrates the preparation of PLA based submicron particle dispersions according to the present invention.

### - The preparation of COMPOSITE-RES-1

A first solution was prepared by dissolving 4.651 g Natureworks Polylactide Resin 4060D in 51.86 g ethyl acetate. A solution of 117 mg NIR-7 in 2 ml dichloromethane was added and the solution was further stirred for 30 minutes.

A second solution was prepared by dissolving 2.33 g Mowiol 4 88 in 93 g water.

The first solution was added to the second solution over 15 minutes, while stirring at 18000 rpm using an Ultra Turrax T25 (IKA). After complete addition, stirring at 18000 rpm was continued for an additional 10 minutes, while keeping the temperature below 32°C.

The solvents ethyl acetate and dichloromethane were removed under reduced pressure at 35°C, while gradually lowering the pressure from 950 mbar to 85 mbar. The weight dispersion was adjusted to 100 g by dilution with water if needed.

The measured average particle size was 282 nm. The dispersion had an absorption maximum at 1036 nm.

### 0.4. Example 2

This example illustrates the preparation of PLGA based composite resin particle dispersions according to the present invention.

### - The preparation of COMPOSITE-RES-2

A first solution was prepared by dissolving 4.651 g Resomer RG503H in 61.86 g ethyl acetate. A solution of 116 mg NIR-7 in 2 ml dichloromethane was added and the solution was further stirred for 30 minutes.

A second solution was prepared by dissolving 4.651 g Synperonic PE/F68-FL in 90.58 g water.

The first solution was added to the second solution over 15 minutes, while stirring at 13000 rpm using an Ultra Turrax T25 (IKA). After complete addition, stirring at 13000 rpm was continued for an additional 10 minutes, while keeping the temperature below 32°C.

The solvents ethyl acetate and dichloromethane were removed under reduced pressure at 35°C, while gradually lowering the pressure from 950 mbar to 85 mbar. The weight dispersion was adjusted to 100 g by dilution with water if needed.

The measured average particle size was 167 nm. The dispersion had an absorption maximum at 1036 nm.

### - The preparation of COMPOSITE-RES-3

A first solution was prepared by dissolving 4.651 g of RESOMER RG503H and 0.116 g NIR 24 in 92 g methylene chloride.

A second solution was prepared by dissolving 2.33 g Mowiol 4 88 in 93 g water.

The first solution was added to the second solution over 15 minutes, while stirring with at 10000 rpm using an Ultra Turrax T25 (IKA). After complete addition, stirring at 10000 rpm was continued for an additional 10 minutes, while keeping the temperature at 21°C.

The solvent was removed under reduced pressure at 35°C, while gradually lowering the pressure from 950 mbar to 85 mbar. The weight of the dispersion was adjusted to 100 g by dilution with water if needed.

The particle size was measured using a light microscope. The average particle size was 2 micron. The dispersion had an absorption maximum at 772 nm.

### - The preparation of COMPOSITE-RES-4 :

A first solution was prepared by dissolving 4.651 g of RESOMER RG503H and 0.116 g NIR 25 in 92 g methylene chloride.

A second solution was prepared by dissolving 2.33 g Mowiol 4 88 in 93 g water.

The first solution was added to the second solution over 15 minutes, while stirring with at 10000 rpm using an Ultra Turrax T25 (IKA). After complete addition, stirring at 10000 rpm was continued for an additional 10 minutes, while keeping the temperature at 21°C.

The solvent was removed under reduced pressure at 35°C, while gradually lowering the pressure from 950 mbar to 85 mbar. The weight of the dispersion was adjusted to 100 g by dilution with water if needed.

The particle size was measured using a light microscope. The average particle size was 2 micron. The dispersion had an absorption maximum at 810 nm.

### 0.5. Example 3:

This example illustrates the NIR response of dispersion comprising NIR responsive submicron particles according to the present invention.

A 1 mm thick coating of diluted dispersions of NIR nanoparticles according to the present invention was exposed to laser radiation on a Coherent laser combination, equipped with 3 lasers, respectively emitting at 920 nm, 1064 nm and 1150 nm. It was evaluated to which extend the different 1 mm thick coating evaporated under laser exposure. The samples were coated on Priplak, a poly(propylene) substrate supplied by Antalis. The results are summarized in Table 2.

**Table 2**

| **Resin particle dispersion** | **Laser response** |
|---|---|
| **COM POSITE-RES-1** | Immediate evaporation over half of the powersweep |
| **COM POSITE-RES-1** | Partial evaporation over the full powersweep |
| **COM POSITE-RES-2** | Immediate full evaporation over the full power sweep |
| **COM POSITE-RES-2** | Immediate full evaporation over the full power sweep |
| **COM POSITE-RES-2** | Immediate full evaporation over the full power sweep |

From Table 2 it becomes apparent that the NIR responsive nanoparticles according to the present invention show high laser response.

## Claims

1. A composite resin particle comprising a NIR absorber according to general formula I and a resin selected from the group consisting of poly(amino acids), polyphosphazenes, polysaccharide derivatives, poly(esters), poly(ortho esters), poly(cyano-acrylates) and copolymers thereof 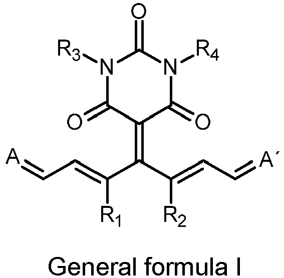
wherein A and A' independently represent a substituted or unsubstituted heterocyclic group, covalently bonded to the polymethine chromophore via a carbon atom
R₁ and R₂ are independently selected from the group consisting of a hydrogen, a substituted or unsubstituted alkyl group, a substituted or unsubstituted alkenyl group, a substituted or unsubstituted alkynyl group, a substituted or unsubstituted aralkyl group, a substituted or unsubstituted alkaryl group and a substituted or unsubstituted aryl or heteroaryl group
R₁ and R₂ may represent the necessary atoms to form a five to eight membered ring
R₃ and R₄ are independently selected from the group consisting of a substituted or unsubstituted alkyl group, a substituted or unsubstituted alkenyl group, a substituted or unsubstituted alkynyl group, a substituted or unsubstituted aralkyl group, a substituted or unsubstituted alkaryl group and a substituted or unsubstituted aryl or heteroaryl group.

2. The composite resin particle according to claim 1 wherein the poly(ester) is selected from the group consisting of a poly(caprolactone), a poly(lactic acid), a poly(L-lactic acid), a poly(glycolic acid), a poly(lactic acid-co-glycolic acid), a poly(L-lactic acid-co-glycolicacid), a poly(D, L-lactide) and any derivative and/or combination thereof.

3. The composite resin particle according to any of the preceding claims wherein the near infrared absorber is according to general formula II 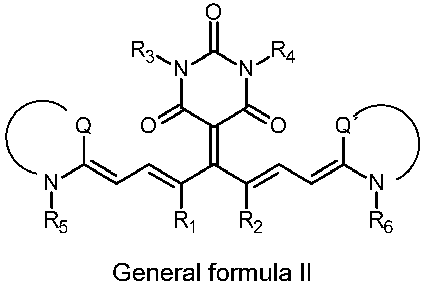 wherein,
R₁ and R₂ are independently selected from the group consisting of a hydrogen, a substituted or unsubstituted alkyl group, a substituted or unsubstituted alkenyl group, a substituted or unsubstituted alkynyl group, a substituted or unsubstituted aralkyl group, a substituted or unsubstituted alkaryl group and a substituted or unsubstituted aryl or heteroaryl group
R₁ and R₂ may represent the necessary atoms to form a five to eight membered ring
R₃ and R₄ are independently selected from the group consisting of a substituted or unsubstituted alkyl group, a substituted or unsubstituted alkenyl group, a substituted or unsubstituted alkynyl group, a substituted or unsubstituted aralkyl group, a substituted or unsubstituted alkaryl group and a substituted or unsubstituted aryl or heteroaryl group
R₅ and R₆ independently represent a substituted or unsubstituted alkyl group, a substituted or unsubstituted alkenyl group, a substituted or unsubstituted alkynyl group, a substituted or unsubstituted aralkyl group, a substituted or unsubstituted alkaryl group and a substituted or unsubstituted aryl or heteroaryl group
Q represents the necessary atoms to form a substituted or unsubstituted five or six membered hetero-ring.

4. The composite resin particle according to any of the preceding claims wherein the NIR absorber is non-ionic.

5. The composite resin particle according to any of the preceding claims wherein the average particle size of the composite resin particles according to the present invention is between 0.03 µm and 3 µm

6. The composite resin particle according to any of the preceding claims further comprising a pharmaceutical active compound.

7. The composite resin particle according to Claim 6 wherein the pharmaceutical active compound is a cytostatics selected from the group consisting of alkylating agents, anthracyclines, cytoskeletal disruptors, epothilones, histone deacetylase inhibitors, topoisomerase I inhibitors, topoisomerase II inhibitors, kinase inhibitors, nucleotide analogs, peptide antibiotics, platinum based agents, retinoids and vinca alkaloids and derivatives.

8. An aqueous dispersion comprising the composite resin particles as defined in claim 1 to 7 and a stabilizing polymer or a surfactant.

9. The aqueous dispersion according to Claim 8 wherein the surfactant is a non-ionic ethoxylated block copolymer surfactant.

10. The aqueous dispersion according to Claim 8 wherein the stabilizing polymer is a polysaccharide, a poly(vinyl alcohol) or a poly(vinyl alcohol) copolymer or derivatives thereof.

11. A pharmaceutical composition comprising the dispersions as defined in claims 8 to10 and a pharmaceutical carrier or excipient.

12. A pharmaceutical composition comprising the dispersion as defined in claims 8 to 10 for use in cancer therapy.

13. A pharmaceutical composition comprising the dispersion as defined in claims 8 to 10 for use in a medical imaging method.

14. A method of preparing an aqueous dispersion as defined in claims 8 to 10 comprising the steps of:
a) dissolving a resin selected from the group consisting of (poly(amino acids), polyphosphazenes, polysaccahride derivatives, poly(esters), poly(ortho esters), poly(cyano-acrylates) and copolymers thereof and a NIR absorber according to general formula I in a substantially water immiscible solvent; and
b) dissolving a dispersant into water; and
c) emulsifying the solution of the resin and the near infrared absorber obtained in step a) into the aqueous solution of the dispersant obtained in step b); and
d) evaporating the substantially water immiscible solvent.

15. The method of preparing the aqueous dispersion according to claim 14 wherein a pharmaceutical active compound is added to the substantially water immiscible solvent in step a).

## Patentansprüche

1. Ein Verbundharzteilchen, enthaltend einen NIR-Absorber gemäß der allgemeinen Formel I und ein Harz, gewählt aus der Gruppe bestehend aus Poly(aminosäuren), Polyphosphazenen, Polysaccharid-Derivaten, Poly(estern), Poly(orthoestern), Poly(cyanoacrylaten) und Copolymeren derselben 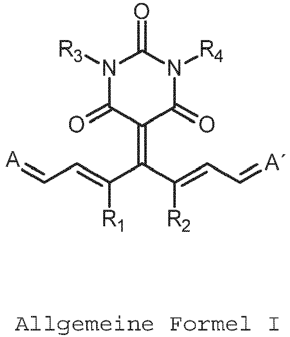
in der A und A' unabhängig voneinander eine substituierte oder nicht-substituierte heterocyclische, über ein Kohlenstoffatom kovalent an den Polymethinchromophor gebundene Gruppe bedeuten, R₁ und R₂ unabhängig voneinander aus der Gruppe bestehend aus einem Wasserstoff, einer substituierten oder nicht-substituierten Alkylgruppe, einer substituierten oder nicht-substituierten Alkenylgruppe, einer substituierten oder nicht-substituierten Alkynylgruppe, einer substituierten oder nicht-substituierten Aralkylgruppe, einer substituierten oder nicht-substituierten Alkarylgruppe und einer substituierten oder nicht-substituierten Aryl- oder Heteroarylgruppe gewählt werden,
R₁ und R₂ die zur Bildung eines fünf- bis achtgliedrigen Ringes benötigten Atome bedeuten können,
R₃ und R₄ unabhängig voneinander aus der Gruppe bestehend aus einer substituierten oder nicht-substituierten Alkylgruppe, einer substituierten oder nicht-substituierten Alkenylgruppe, einer substituierten oder nicht-substituierten Alkynylgruppe, einer substituierten oder nicht-substituierten Aralkylgruppe, einer substituierten oder nicht-substituierten Alkarylgruppe und einer substituierten oder nicht-substituierten Aryl- oder Heteroarylgruppe gewählt werden.

2. Das Verbundharzteilchen nach Anspruch 1, wobei der Poly(ester) aus der Gruppe bestehend aus einem Poly(caprolacton), einer Poly(milchsäure), einer Poly(L-milchsäure), einer Poly(glycolsäure), einer Poly(milchsäure-co-glycolsäure), einer Poly(L-milchsäure-co-glycolsäure), einem Poly(D,L-lactid) und einem beliebigen Derivat und/oder einer Kombination derselben gewählt wird.

3. Das Verbundharzteilchen nach einem der vorstehenden Ansprüche, wobei der NIR-Absorber der allgemeinen Formel II entspricht: 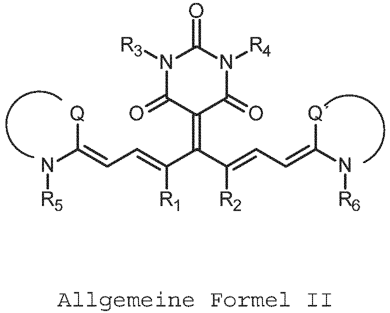 in der
R₁ und R₂ unabhängig voneinander aus der Gruppe bestehend aus einem Wasserstoff, einer substituierten oder nicht-substituierten Alkylgruppe, einer substituierten oder nicht-substituierten Alkenylgruppe, einer substituierten oder nicht-substituierten Alkynylgruppe, einer substituierten oder nicht-substituierten Aralkylgruppe, einer substituierten oder nicht-substituierten Alkarylgruppe und einer substituierten oder nicht-substituierten Aryl- oder Heteroarylgruppe gewählt werden,
R₁ und R₂ die zur Bildung eines fünf- bis achtgliedrigen Ringes benötigten Atome bedeuten können,
R₃ und R₄ unabhängig voneinander aus der Gruppe bestehend aus einer substituierten oder nicht-substituierten Alkylgruppe, einer substituierten oder nicht-substituierten Alkenylgruppe, einer substituierten oder nicht-substituierten Alkynylgruppe, einer substituierten oder nicht-substituierten Aralkylgruppe, einer substituierten oder nicht-substituierten Alkarylgruppe und einer substituierten oder nicht-substituierten Aryl- oder Heteroarylgruppe gewählt werden,
R₅ und R₆ unabhängig voneinander eine substituierte oder nicht-substituierte Alkylgruppe, eine substituierte oder nicht-substituierte Alkenylgruppe, eine substituierte oder nicht-substituierte Alkynylgruppe, eine substituierte oder nicht-substituierte Aralkylgruppe, eine substituierte oder nicht-substituierte Alkarylgruppe und eine substituierte oder nicht-substituierte Aryl- oder Heteroarylgruppe bedeuten,
Q die zur Bildung eines substituierten oder nicht-substituierten fünf- oder sechsgliedrigen Ringes benötigten Atome bedeutet.

4. Das Verbundharzteilchen nach einem der vorstehenden Ansprüche, wobei der NIR-Absorber nicht-ionisch ist.

5. Das Verbundharzteilchen nach einem der vorstehenden Ansprüche, wobei die mittlere Teilchengröße der Verbundharzteilchen gemäß der vorliegenden Erfindung zwischen 0,03 µm und 3 µm liegt.

6. Das Verbundharzteilchen nach einem der vorstehenden Ansprüche, das ferner eine aktive pharmazeutische Verbindung enthält.

7. Das Verbundharzteilchen nach Anspruch 6, wobei die aktive pharmazeutische Verbindung ein Zytostatikum ist, das aus der Gruppe bestehend aus Alkylierungsmitteln, Anthracyclinen, Zytoskelett-Disruptoren, Epothilonen, Histondeacetylashemmern, Topoisomerase I-Hemmern, Topoisomerase II-Hemmern, Kinasehemmern, Nukleotidanaloga, Peptid-Antibiotika, Mitteln auf Platinbasis, Retinoiden und Vinca-Alkaloiden und Derivaten derselben gewählt wird.

8. Eine wässrige Dispersion, enthaltend die wie im Anspruch 1 bis 7 definierten Verbundharzteilchen und ein stabilisierendes Polymer oder ein Tensid.

9. Die wässrige Dispersion nach Anspruch 8, wobei das Tensid ein nicht-ionisches ethoxyliertes Blockcopolymer-Tensid ist.

10. Die wässrige Dispersion nach Anspruch 8, wobei das stabilisierende Polymer ein Polysaccharid, ein Poly(vinylalkohol) oder ein Poly(vinylalkohol)-Copolymer oder ein Derivat derselben ist.

11. Eine pharmazeutische Zusammensetzung, die die wie nach den Ansprüchen 8 bis 10 definierten Dispersionen und einen pharmazeutischen Träger oder Hilfsstoff enthält.

12. Eine pharmazeutische Zusammensetzung, die die wie nach den Ansprüchen 8 bis 10 definierte Dispersion zur Verwendung in einer Krebstherapie enthält.

13. Eine pharmazeutische Zusammensetzung, die die wie nach den Ansprüchen 8 bis 10 definierte Dispersion zur Verwendung in einem medizinischen Bildgebungsverfahren enthält.

14. Ein Verfahren zur Herstellung einer wie nach den Ansprüchen 8 bis 10 definierten wässrigen Dispersion, das die folgenden Schritte umfasst:
a) Auflösen eines Harzes, das aus der Gruppe bestehend aus Poly(aminosäuren), Polyphosphazenen, Polysaccharid-Derivaten, Poly(estern), Poly(orthoestern), Poly(cyanoacrylaten) und Copolymeren derselben gewählt wird, und eines NIR-Absorbers gemäß der allgemeinen Formel I in einem mit Wasser wesentlich unmischbaren Lösungsmttel, und
b) Auflösen eines Dispergiermittels in Wasser, und
c) Emulgieren der im Schritt a) erhaltenen Lösung des Harzes und des NIR-Absorbers in der im Schritt b) erhaltenen wässrigen Lösung des Dispergiermittels, und
d) Abdampfen des mit Wasser wesentlich unmischbaren Lösungsmittels.

15. Das Verfahren zur Herstellung der wässigen Dispersion nach Anspruch 14, wobei dem mit Wasser wesentlich unmischbaren Lösungsmittel im Schritt a) eine aktive pharmazeutische Verbindung zugesetzt wird.

## Revendications

1. Particule de résine composite contenant un agent absorbant le rayonnement infrarouge proche répondant à la formule générale I et une résine choisie parmi le groupe composé d'acide poly(aminé), de polyphosphazènes, de dérivés de polysaccharide, de poly(esters), de poly(orthoesters), de poly(cyanoacrylates) et de copolymères de ceux-ci 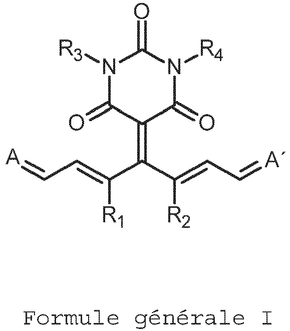
où A et A' représentent, l'un indépendamment de l'autre, un groupe hétérocyclique, substitué ou non substitué et lié par covalence au chromophore de polyméthine par un atome de carbone, R₁ et R₂ sont choisis, l'un indépendamment de l'autre, parmi le groupe composé d'un hydrogène, d'un groupe alkyle substitué ou non substitué, d'un groupe alkényle substitué ou non substitué, d'un groupe alkynyle substitué ou non substitué, d'un groupe aralkyle substitué ou non substitué, d'un groupe alcaryle substitué ou non substitué et d'un groupe aryle ou hétéroaryle substitué ou non substitué,
R₁ et R₂ peuvent représenter les atomes nécessaires pour former un cycle à cinq à huit membres,
R₃ et R₄ sont choisis, l'un indépendamment de l'autre, parmi le groupe composé d'un groupe alkyle substitué ou non substitué, d'un groupe alkényle substitué ou non substitué, d'un groupe alkynyle substitué ou non substitué, d'un groupe aralkyle substitué ou non substitué, d'un groupe alcaryle substitué ou non substitué et d'un groupe aryle ou hétéroaryle substitué ou non substitué.

2. Particule de résine composite selon la revendication 1, **caractérisée en ce que** le poly(ester) est choisi parmi le groupe composé d'une poly(caprolactone), d'un acide poly(lactique), d'un acide poly(L-lactique), d'un acide poly(glycolique), d'un acide poly(lactique-co-glycolique), d'un acide poly(L-lactique-co-glycolique), de poly(D,L-lactide) et d'un dérivé quelconque et/ou d'une combinaison de ceux-ci.

3. Particule de résine composite selon l'une quelconque des revendications précédentes, **caractérisée en ce que** l'agent absorbant le rayonnement infrarouge proche répond à la formule générale II: 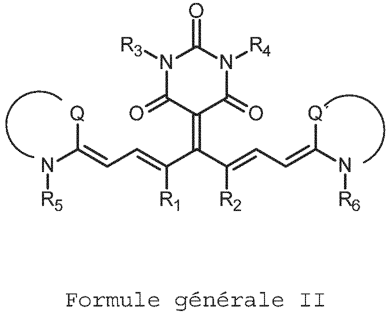 où
R₁ et R₂ sont choisis, l'un indépendamment de l'autre, parmi le groupe composé d'un hydrogène, d'un groupe alkyle substitué ou non substitué, d'un groupe alkényle substitué ou non substitué, d'un groupe alkynyle substitué ou non substitué, d'un groupe aralkyle substitué ou non substitué, d'un groupe alcaryle substitué ou non substitué et d'un groupe aryle ou hétéroaryle substitué ou non substitué,
R₁ et R₂ peuvent représenter les atomes nécessaires pour former un cycle à cinq à huit membres,
R₃ et R₄ sont choisis, l'un indépendamment de l'autre, parmi le groupe composé d'un groupe alkyle substitué ou non substitué, d'un groupe alkényle substitué ou non substitué, d'un groupe alkynyle substitué ou non substitué, d'un groupe aralkyle substitué ou non substitué, d'un groupe alcaryle substitué ou non substitué et d'un groupe aryle ou hétéroaryle substitué ou non substitué,
R₅ et R₆ représentent, l'un indépendamment de l'autre, un groupe alkyle substitué ou non substitué, un groupe alkényle substitué ou non substitué, un groupe alkynyle substitué ou non substitué, un groupe aralkyle substitué ou non substitué, un groupe alcaryle substitué ou non substitué et un groupe aryle ou hétéroaryle substitué ou non substitué,
Q représente les atomes nécessaires pour former un cycle à cinq ou six membres substitué ou non substitué.

4. Particule de résine composite selon l'une quelconque des revendications précédentes, **caractérisée en ce que** l'agent absorbant le rayonnement infrarouge proche est non ionique.

5. Particule de résine composite selon l'une quelconque des revendications précédentes, **caractérisée en ce que** la taille de particule moyenne des particules de résine composite selon la présente invention est comprise entre 0,03 µm et 3 µm.

6. Particule de résine composite selon l'une quelconque des revendications précédentes contenant en outre un composé pharmaceutique actif.

7. Particule de résine composite selon la revendication 6, **caractérisée en ce que** le composé pharmaceutique actif est un cytostatique choisi parmi le groupe composé d'agents alkylants, d'anthracyclines, de perturbateurs de cytosquelette, d'épothilones, d'inhibiteurs d'histone désacetylases, d'inhibiteurs de la topoisomérase I, d'inhibiteurs de la topoisomérase II, d'inhibiteurs de la kinase, d'analogues des nucléotides, de peptides antibiotiques, d'agents à base de platine, de rétinoïdes et de vinca-alcaloïdes et de dérivés de ceux-ci.

8. Dispersion aqueuse contenant les particules de résine composite telles que définies selon la revendication 1 à 7 et un polymère stabilisant ou un tensioactif.

9. Dispersion aqueuse selon la revendication 8, **caractérisée en ce que** le tensioactif est un tensioactif d'un copolymère à blocs éthoxylé non ionique.

10. Dispersion aqueuse selon la revendication 8, **caractérisée en ce que** le polymère stabilisant est un polysaccharide, un alcool poly(vinylique) ou un copolymère d'alcool poly(vinylique) ou des dérivés de ceux-ci.

11. Composition pharmaceutique contenant les dispersions telles que définies selon les revendications 8 à 10 et un véhicule ou excipient pharmaceutique.

12. Composition pharmaceutique contenant la dispersion telle que définie selon les revendications 8 à 10 pour être utilisée dans une thérapie antitumorale.

13. Composition pharmaceutique contenant la dispersion telle que définie selon les revendications 8 à 10 pour être utilisée dans un procédé d'imagerie médicale.

14. Procédé pour la préparation d'une dispersion aqueuse telle que définie selon les revendications 8 à 10 comprenant les étapes consistant à:
a) dissoudre une résine choisie parmi le groupe composé d'acide poly(aminé), de polyphosphazènes, de dérivés de polysaccharide, de poly(esters), de poly(orthoesters), de poly(cyanoacrylates) et de copolymères de ceux-ci, et un agent absorbant le rayonnement infrarouge proche répondant à la formule générale I dans un solvant essentiellement immiscible à l'eau, et
b) dissoudre un dispersant dans de l'eau, et
c) émulsionner la solution de la résine et de l'agent absorbant le rayonnement infrarouge proche obtenue dans l'étape a) dans la solution aqueuse du dispersant obtenue dans l'étape b), et
d) faire évaporer le solvant essentiellement immiscible à l'eau.

15. Procédé pour la préparation de la dispersion aqueuse selon la revendication 14, **caractérisé en ce que**, dans l'étape a), il est ajouté un composé pharmaceutique actif au solvant essentiellement immiscible à l'eau.
